# EUROPÄISCHE PATENTSCHRIFT

(11) **EP 1 882 476 B1**
(45) Veröffentlichungstag und Bekanntmachung des Hinweises auf die Patenterteilung: **19.11.2008**
(21) Anmeldenummer: 06015520.7
(22) Anmeldetag: 25.07.2006
(51) Int. Cl.: A61K 31/549, A61P 31/00

(54) **Herstellung von antimikrobiellen Formulierungen unter Verwendung von 7-Oxa-2-thia 1,5-diazabicyclo[3.3.1]nonan-2,2-dion**
Preparation of antimicrobial formulation comprising 7-oxo-2-thia-1,5-diazabicyclo[3.3.1]-nonan-2,2-dion
Production d' une préparation antimicrobienne contenant 7-oxo-2-thia-1,5-diazabicyclo[3.3.1]-nonan-2,2-dion

(43) Veröffentlichungstag der Anmeldung: 30.01.2008
(73) Patentinhaber: HERDEIS, Claus, 80809 München (DE); Weis, Christian Edwin, 97297 Waldbüttelbrunn (DE)
(72) Erfinder: HERDEIS, Claus, 80809 München (DE); Weis, Christian Edwin, 97297 Waldbüttelbrunn (DE)
(74) Vertreter: Andrae, Steffen

(56) Entgegenhaltungen:
- WO-A-00/01391
- DE-C1- 19 708 782
- JUREWITSCH B ET AL: "Taurolidine lock: The key to prevention of recurrent catheter-related bloodstream infections" CLINICAL NUTRITION, CHURCHILL LIVINGSTONE, LONDON, GB, Bd. 24, Nr. 3, Juni 2005 (2005-06), Seiten 462-465, XP004893209 ISSN: 0261-5614
- KENNEDY, ALAN R. ET AL: "Two new compounds by reaction of taurolidine with methylene glycol" ACTA CRYSTALLOGRAPHICA, SECTION C: CRYSTAL STRUCTURE COMMUNICATIONS , C55(2), 232-234 CODEN: ACSCEE; ISSN: 0108-2701, 1999, XP009076104

## Beschreibung

Die vorliegende Erfindung betrifft das Gebiet antimikrobieller (antiseptischer bzw. mikrobizider) Formulierungen für medizinische und andere Zwecke, wobei die Formulierungen insbesondere wässrige Lösungen sind, die einen Wirkstoff enthalten, dessen antimikrobielle und antiendotoxische Wirksamkeit mit einem irreversiblen N-Methylolgruppen-Transfer auf die mikrobiellen Zellwände sowie Endotoxine erklärt wird.

Als Wirkstoff, dessen Wirksamkeit im Sinne des genannten Methylolgruppentransfers gedeutet wird, ist im wesentlichen die Verbindung Taurolidin zu nennen. Taurolidin ist eine Substanz, die sich strukturmäßig von der Aminosulfonsäure Taurin ableiten läßt. Taurolidin weist die Strukturformel (I) auf und steht in wässriger Lösung im Gleichgewicht mit Taurultam (II) und Methyloltaurultam (III).

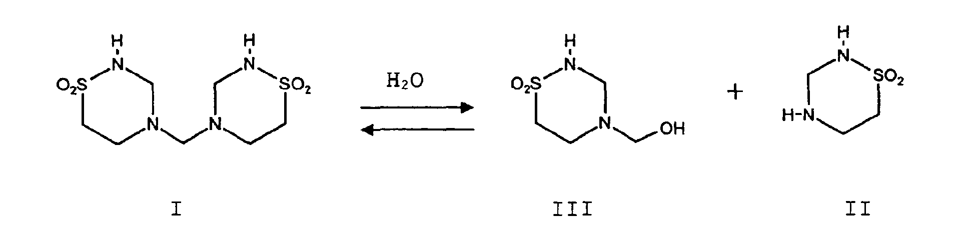

Die Herstellung von Taurolidin wurde erstmals beschrieben in der Schweizer Patentveröffenlichung CH 482713 A. Taurolidin wird hergestellt durch Umsetzung von Taurinamid (2-Aminoethansulfonsäureamid) mit 1,5 Äquivalenten Formaldehyd bzw. seinem in wässriger Lösung gebildeten Hydratisierungsprodukt Methylenglykol CH₂(OH)₂. Das bekannte Verfahren wurde durch ein verbessertes Verfahren zur Herstellung von Taurinamid gemäß EP 0 863 133 B19 erheblich verbessert.

Die Bildung von Taurolidin aus Taurinamid und Formaldehyd läßt sich beschreiben als Kondensationsprozess, an dem 2 Äquivalente Taurinamid und 3 Äquivalente Formaldehyd beteiligt sind, was für die Gesamtverbindung Taurolidin ein Verhältnis von Taurinamid zu Formaldehyd von 1:1,5 ergibt. Bei der hydrolytischen Spaltung von Taurolidin gemäß der obigen Formelgleichung bilden sich die Verbindungen Methyloltaurultam und Taurultam, die, einzeln betrachtet, als Kondensationsprodukte von Taurinamid mit 2 Äquivalenten bzw. 1 Äquivalent Formaldehyd angesehen werden können.

Taurolidin ist seit langem in Form wässriger Lösungen unter der Marke Taurolin^{®} im Handel, und zwar als Taurolin 2% Instillationslösung mit einem Gehalt von 2,0 g Taurolidin und 5,0 g Polyvinylpyrrolidon pro 100 ml Wasser, sowie als Taurolin^{®} Ringer 0,5% chirurgische Spüllösung mit 0,50 g Taurolidin, 1,25 g Polyvinylpyrrolidon sowie einer Mischung anorganischer Salze pro 100 ml Wasser.

Die medizinischen Verwendungen der Taurolin^{®}-Lösungen beruhen im wesentlichen auf der antimikrobiellen und antiendotoxischen Wirksamkeit von Taurolidin. Die 0,5%-ige Taurolinlösung dient vor allem zur intraoperativen Spülung der Bauchhöhle, während für die 2%-igen Taurolin^{®}-Lösungen Anwendungen per Instillation über die Dränagen insbesondere bei diffuser eitriger Peritonitis und Appendicitis perforata angegeben werden. Andere vom Hersteller angegebene Anwendungen sind die Prophylaxe bei Weichteil- und Knochenverletzungen und bei Pleuraemphem. In verschiedenen Patentanmeldungen und wissenschaftlichen Veröffentlichungen werden weitere Anwendungsmöglichkeiten von Taurolin^{®}, beispielsweise in der Zahnmedizin und bei der Oralhygiene, bei Tumorerkrankungen sowie in der Dermatologie beschrieben, und es wurde die Beeinflussung einer großen Zahl von physiologischen Parametern durch lokale oder parenterale Verabreichung von Taurolin^{®}-Lösungen bzw. Taurolidinlösungen untersucht.

Es ist ferner bereits seit 1989 bekannt, dass die bekannten Taurolin^{®}-Lösungen auch dazu verwendet werden können, die sogenannte "Kathetersepsis" zu bekämpfen (vgl. Mughal, Br. J. Cir.(1989) 76(1), Seiten 15 bis 21; vgl. auch J. of the Critically I11 (1990) 6 (6), Seiten 228 bis 231). "Kathetersepsis" ist einer der Begriffe für schwere Komplikationen, die bei Personen auftreten können, denen zur wiederholten Zufuhr von Medikamenten oder Nährlösungen oder zu Hämodialysezwecken Katheter implantiert sind. Als "Katheter" können auch die sogenannten Port-Systeme angesehen werden, die ebenfalls dauerhaft implantiert sind und einen externen Zugang zu zentralen Blutgefäßen eines Patienten ermöglichen. Kommt es während des auf Dauer angelegten Gebrauchs der Katheter oder Port-Systeme zu einer Besiedlung dieser Katheter oder Port-Systeme mit pathogenen Bakterien, beispielsweise im Sinne einer Bildung von Biofilmen an den Innenwänden der Katheter oder Port-Systeme oder den zugehörigen Leitungswegen wie z.B. Hohlnadeln, kann es bei dem Patienten zu gefährlichen lokalen und insbesondere systemischen Infektionen (Sepsis) kommen.

Es ist daher seit langem grundsätzlich bekannt, Katheter und Port-Systeme während derjenigen Zeiten, in denen z.B. keine Zuführung von Medikamenten oder Nährlösungen erfolgt oder kein Blut abgenommen wird, mit antikmikrobiell wirksamen Lösungen zu füllen, die eine Besiedelung der Katheter und Port-Systeme durch Mikroorganismen und die Bildung von nur schwer mit Antibiotika oder Antiseptika bekämpfbaren Biofilmen verhindern.

In der europäischen Patentanmeldung EP 0 946 221 A1 wird die Verwendung einer Taurolin^{®}-Lösung als Locklösung beschrieben, wobei vorgesehen ist, dass die Locklösung vor einer Wiederinbetriebnahme des Katheters mit einer Salzlösung in den Blutstrom gespült wird. Da die Locklösung bei einem derartigen Vorgehen in den Patientenorganismus gelangt, muß die Locklösung alle Voraussetzungen erfüllen, die an parenteral zu verabreichende Arzneimittel gestellt werden.

Während in EP 0 946 221 A1 im wesentlichen die Verwendung der üblichen 2 Gew.-%igen Taurolin^{®}-Lösungen, die neben Taurolidin Polyvinylpyrrolidon enthalten, beschrieben wird, wird im europäischen Patent EP 1 089 738 B1 eine Verwendung von Taurolidin für Locklösungen in modifizierter Form offenbart, und zwar als Lösung von Taurolidin in einem Puffersystem aus Trinatriumcitrat und Citronensäure. Das Puffersystem dient einer Optimierung der Löslichkeit des Taurolidins und seiner antibakteriellen Wirksamkeit, wobei außerdem die koagulationshemmenden Eigenschaften von Citrat dazu genutzt werden, eine Blockierung der Katheterausgänge durch Koagulate, die aus Blut gebildet werden können, zu verhindern. Locklösungen der in EP 1 089 738 B1 beschriebenen Art werden heute in der Regel nicht mehr in die Zirkulation gespült, sondern vor der Wiederinbetriebnahme des Katheters oder Port-Systems aus diesem abgesaugt. Das hat unter anderem den Vorteil, dass die Locklösungen nicht in den Körper des Patienten gelangen und daher nicht als Therapeutika angesehen werden, die die strengen Zulassungsvoraussetzungen für Therapeutika erfüllen müssen, sondern als nur äußerlich einwirkende Desinfektionsmittel bzw. Antiseptika.

Obwohl die Verwendung von Taurolidin in Locklösungen, insbesondere in Locklösungen mit Citratzusatz, z.B. im Vergleich mit Lösungen von Antibiotika, eine entscheidende Verbesserung im Hinblick auf die Bekämpfung von Infektionen bedeutete, die durch eine mikrobielle Kontamination von Kathedern und Port-Systemen ausgelöst werden, sind auf dem genannten Gebiet noch Verbesserungen möglich. Obwohl Taurolidinlösungen gegen ein außerordentlich breites Spektrum von Bakterien (prokaryontischen Mikroorganismen) wirksam sind, ist ihre Wirksamkeit gegenüber eukaryontischen Mikroorganismen (Fungi; z.B. Hefen oder Schimmelpilzen) beschränkt.

Aufgrund der begrenzten Löslichkeit von Taurolidin in wässrigen Medien kann die Wirksamkeit wässriger antimikrobieller Lösungen auf Taurolidinbasis nicht beliebig durch Konzentrationserhöhungen verstärkt werden. Diese Beobachtung betrifft nicht nur Locklösungen, sondern generell mikrobizide (antimikrobielle; antiseptische) wässrige Lösungen auf Taurolidinbasis auch für andere bekannte oder denkbare Verwendungszwecke.

Es ist Aufgabe der vorliegenden Erfindung, eine neuartige Herstellung von Formulierungen mit einer antimikrobiellen Wirksamkeit, die auf einem Methylolgruppentransfer von der/den in der Formulierung enthaltenen Spezies auf Mikroorganismen beruht, zu schaffen, das es ermöglicht, im genannten Sinne wirksame Formulierungen zu schaffen, deren Wirksamkeit gegenüber Mikroorganismen, insbesondere gegenüber eukaryontischen Mikroorganismen, im Vergleich mit bekannten Taurolidinlösungen erhöht ist, die es dabei jedoch gleichzeitig ermöglichen, die gewünschte Wirksamkeit zu modifizieren und z.B. ganz an die der bisher bekannten Taurolidinlösungen, insbesondere Taurolidin-Locklösungen, anzupassen, wenn eine höhere Wirksamkeit nicht benötigt wird oder im konkreten Einzelfall aufgrund unerwünschter Nebeneffekte vermieden werden soll.

Diese Aufgabe wird gemäß der vorliegenden Erfindung dadurch gelöst, dass man zur Herstellung der antimikrobiell wirkenden Formulierungen nicht Taurolidin verwendet, sondern die Verbindung 7-Oxa-2-thia-1,5-diazabicyclo[3.3.1]nonan-2,2-dion, die in der weiteren Anmeldung vereinfacht als "Cyclotaurolidin" bezeichnet wird.

Die vorliegende Erfindung betrifft somit generell die Verwendung von Cyclotaurolidin zur Herstellung von antimikrobiell (mikrobiziden) oder antiseptisch wirksamen Formulierungen für beliebige Zwecke. Diese Zwecke umfassen neben Verwendungen für medizinische Zwecke, insbesondere Verwendungen als wässrige Locklösungen, auch Verwendungen für andere technische Zwecke, z.B. in Mitteln zur Oberflächensterilisation von Körperpartien oder Gegenständen, die sterile Oberflächen aufweisen sollen, beispielsweise von Kontaktlinsen, Implantaten (z.B. stents) und anderen Instrumenten, die so verwendet werden, dass sie Infektionen auslösen können. Die genannte Verwendung kann auch als neues Verfahren zur Herstellung von Lösungen ausgehend von dem neuen Ausgangsprodukt Cyclotaurolidin formuliert werden.

Die Formulierungen können nicht nur Lösungen sein, sondern auch Gele und Salben in für den jeweiligen Verwendungszweck optimierten Trägern.

Die Erfindung betrifft ferner die bei der Verwendung von Cyclotaurolidin zur Herstellung wässriger Lösung erhaltenen Lösungen selbst, insbesondere in der Ausgestaltung als Locklösungen für Katheter und Port-Systeme, und die Verwendung derartiger aus Cyclotaurolidin hergestellten Lösungen als Locklösungen in Kathedern und Port-Systemen.

Ferner ist Gegenstand der Erfindung ein neues, von den Erfindern entwickeltes Verfahren, das die Herstellung von Cyclotaurolidin auf einfache Weise in hohen Ausbeuten ermöglicht.

Die hierin als Cyclotaurolidin bezeichnete Verbindung ist an sich bekannt. In der Veröffentlichung von Alan R. Kennedy et al. "Two new compounds by reaction of taurolidin with methylene glycol", Acta Cryst. (1999). C55, 232-234 wird angegeben, dass diese Verbindung erhalten wird, wenn man Taurolidin mit einem Überschuss Methylenglykol (wässriger Formaldehyd) umsetzt. Die genannte Verbindung weist die Strukturformel (IV) auf:

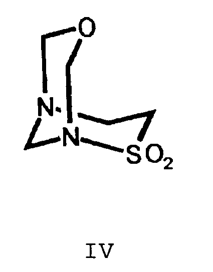

Strukturell gesehen kann diese Verbindung als Taurultam angesehen werden, dessen beide Ringstickstoffatome durch eine Dimethylenätherbrücke unter Ausbildung eines Bicyclus verbunden sind. Die Verbindung kann auch als Produkt einer Kondensationsreaktion von Taurinamid mit 3 Äquivalenten Methylenglykol bzw. Formaldehyd angesehen werden. 1 Äquivalent Methylenglykol bildet die CH₂-Gruppe, die auch im Taurultam vorhanden ist. Aus zwei weiteren Taurultam-N-gebundenen Methylenglykoleinheiten wird in einer nachfolgenden Kondensationsreaktion die sauerstoffhaltige Brücke gebildet.

Die genannte Verbindung wurde als schwer lösliches Nebenprodukt auffällig, das unter bestimmten Bedingungen unerwünscht aus Taurolidinlösungen auskristallisiert. Da es durch Umsetzung des als Wirkstoff wertvollen Taurolidins mit weiterem Methylenglykol bzw. Formaldehyd erhalten wurde, dürfte seine gezielte Herstellung bisher nicht attraktiv erschienen sein, zumal die Verbindung lipophiler ist als Taurolidin. Ferner wurden keinerlei Daten zur antimikrobiellen Wirksamkeit der Verbindung bekannt.

Untersuchungen der Erfinder der vorliegenden Anmeldung zur Löslichkeit und antimikrobiellen Wirksamkeit von Cyclotaurolidin führten nunmehr jedoch zu dem überraschenden Ergebnis, dass wässrige Lösungen von Cyclotaurolidin trotz der höheren Lipophilie der Verbindung nicht nur im Hinblick auf ihre antimikrobielle Wirksamkeit gegen prokaryontische Mikroorganismen (Bakterien) den üblichen höher konzentrierten Taurolidinlösungen gleichwertig sind, sondern dass die wässrigen Lösungen von Cyclotaurolidin außerdem gegenüber problematischen eukaryontischen Mikroorganismen (Hefen, Schimmelpilzen) deutlich wirksamer sind als höher konzentrierte wässrige Lösungen, die durch Auflösen von Taurolidin hergestellt wurden.

In wässrigen Lösungen von Cyclotaurolidin liegt ein Wirkstoff vor, ggf. auch in Form seiner hydrolytisch gebildeten Gleichgewichtsprodukte, bei dem das Molverhältnis von Struktureinheiten, die sich von Taurinamid ableiten, zu Struktureinheiten, die sich von Methylenglykol bzw. Formaldehyd ableiten (Methylengruppen zwischen Ringstickstoffatomen oder zwischen Stickstoff- und Sauerstoffatomen) 1:3 beträgt, während das entsprechende Verhältnis in Lösungen, die unter Verwendung von Taurolidin hergestellt wurden, 1:1,5 beträgt. Dieser Befund kann als Erklärung dafür dienen, dass Lösungen von Cyclotaurolidin, die nur halb so konzentriert sind wie oder sogar geringer konzentriert sind als Taurolidinlösungen, mindestens vergleichbare antimikrobielle Wirksamkeiten zeigen.

Damit weisen durch Auflösen von Cyclotaurolidin hergestellte antimikrobielle wässrige Lösungen gegenüber Lösungen von Taurolidin verschiedene Vorteile auf, die beispielsweise für Anwendungsfälle wichtig sind, bei denen eine höhere Wirksamkeit gegen eukaryontische Mikroorganismen (Hefen, Schimmelpilze) erforderlich ist als die der üblichen Taurolidinlösungen. Eine solche Anwendungsmöglichkeit beinhaltet z.B. eine fungizide Lösung für medizinische bzw. Hygienezwecke, z.B. zur Behandlung der Hautoberfläche. Auch Anwendungen als Konservierungsmittel für technische, kosmetische und Nahrungsmittel-Zwecke erscheinen möglich.

In Fällen, in denen eine erhöhte Wirksamkeit der unter Verwendung von Cyclotaurolidin hergestellten wässrigen Lösungen nicht erforderlich ist oder die höhere Reaktivität im jeweiligen Verwendungsfall auf Bedenken stößt, kann die Aktivität der wässrigen Cyclotaurolidinlösungen durch Zugabe von Taurinamid bzw. Taurinamidhydrochlorid maßgeschneidert verändert werden. Durch Zusatz von Taurinamid läßt sich das Verhältnis von Methylengruppen aus Methylenglykol/Formaldehyd zu Taurinamid kontinuierlich verschieben. Dadurch ist eine Modifikation der antimikrobiellen Wirksamkeit der wässrigen Lösungen sowie auch der Löslichkeit der in der wässrigen Lösung enthaltenen Anteile des Wirkstoffs bzw. seiner Gleichgewichts-Hydrolyseprodukte möglich. Wird der Cyclotaurolidin-Lösung so viel Taurinamid bzw. Taurinamidhydrochlorid zugesetzt, dass das Verhältnis von Methylengruppen, die sich von Methylenglykol ableiten, zu Taurinamid von dem Verhältnis 3:1, wie es im Cyclotaurolidin vorliegt, auf 1,5:1, wie es in Taurolidinlösungen vorliegt (Zusatz von 1 Äquivalent Taurinamid pro Äquivalent Cyclotaurolidin), vermindert wird, verändert sich die antimikrobielle Wirksamkeit der Lösung so, dass Lösungen erhalten werden, die die gleiche antimikrobielle Wirksamkeit aufweisen wie Taurolidinlösungen. Wird zur Aktivitätseinstellung 1 Äquivalent Taurinamidhydrochlorid verwendet, unterscheiden sich die erhaltenen Lösungen von solchen, die duch Auflösen von Taurolidin erhalten wurden, durch die Anwesenheit von Chlorid.

Bei einem entsprechenden Verhältnis von Taurinamid zu Methylenglykol von 2:1 entspricht die Stöchiometrie der Mischungsbestandteile der Stöchiometrie der Verbindung (V), 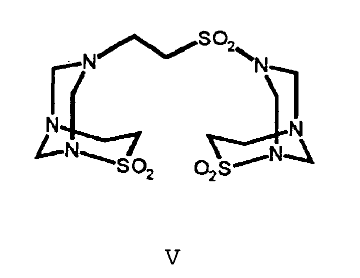 die ebenfalls in der bereits genannten Veröffentlichung in Acta Cryst. (1999). C55, 232-234 beschrieben wird. Es ist davon auszugehen, dass wässrige Lösungen, die durch Auflösen der genannten Verbindung (V) gebildet werden, ebenfalls eine antimikrobielle Wirksamkeit aufweisen, die in etwa die Zahl der Methylenbrücken zwischen Ring-Stickstoffatomen in (V) widerspiegelt. Wässrige Lösungen, die durch Auflösen der genannten Verbindung (V) hergestellt werden, können als wässrige Lösungen von Cyclotaurolidin betrachtet werden, die durch Taurinamidzugabe entsprechent modifiziert wurden und liegen daher ebenfalls im Bereich der vorliegenden Erfindung.

Nachfolgend wird die vorliegende Erfindung anhand von Beispielen näher erläutert, die einerseits ein neues und vorteilhaftes Verfahren zur Herstellung von Cyclotaurolidin beschreiben, andererseits Ergebnisse, die bei der Prüfung der antimikrobiellen Wirksamkeit von wässrigen Lösungen, die aus Cyclotaurolidin hergestellt wurden, wiedergeben.

### Beispiele:

### 1. Herstellung von 7-Oxa-2-thia-1,5-diazabicyclo[3.3.1]nonan-2,2-dion ("Cyclotaurolidin") ausgehend von Taurinamidhydrochlorid.

Taurinamidhydrochlorid (250,0 g, 1,55 Mol; hergestellt gemäß EP 863 133 B19) wurde zu einer Lösung von Natriumhydroxid (60,0 g, 1,50 Mol) in Wasser (700 ml) gegeben. Es wurde eine klare farblose Lösung erhalten, die tropfenweise innerhalb eines Zeitraums von 5 min zu einer gerührten wässrigen Lösung von Formaldehyd (35%, 1000 ml) zugegeben wurde. Während der Zugabe stieg die Temperatur der klaren farblosen Mischung von Raumtemperatur auf 47°C an. Beim Erhitzen der erhaltenen Lösung auf 62°C trübte sich die Lösung ein. Sie wurde weitere 30 min bei einer Temperatur von etwa 45-47°C gerührt und anschließend auf 10°C abgekühlt und bei dieser Temperatur weitere 20 min gerührt. Es bildete sich ein farbloser Niederschlag, der unter Absaugen von der flüssigen Phase abgetrennt wurde, und der mit Wasser und anschließend Ethanol gewaschen und anschließend getrocknet wurde. Die Ausbeute betrug 224,8 g, der Schmelzpunkt des kristallinen Produkts betrug 148,5 g.

### Analyse:

Gefunden: C 33,79, H 5,593, N 15,68, S 18,11
Berechnet (für C₅H₁₀N₂O₃S - Molare Masse 178,21 g):
C 33,69, H 5,65, N 15,71, S 17,99.
IR: ν (cm⁻¹) = 2872, 1384, 1329, 1269, 1225, 1185, 1138, 1064, 1014, 995, 973, 947, 874, 791, 743, 663, 615.
¹³C NMR (CHCl₃) : δ (ppm) = 48,8, 51,5, 69, 0, 79,2, 84,0

Das Produkt ist ein geruchloses, kristallines Pulver, das aus z.B. Ethanol umkristallisiert und gereinigt werden kann, das an der Luft bei üblichen Temperaturen stabil ist, gut gehandhabt werden kann und das nicht nur in Wasser die o.g. Löslichkeit aufweist, sondern auch in verschiedenen organischen Lösemitteln löslich ist. Anders als Taurolidin kann Cyclotaurolidin daher auch zu Formulierungen in organischen Trägern verarbeitet werden, z.B. in vielen üblichen organischen Trägermaterialien für pharmazeutische und kosmetische Zwecke. Das ermöglicht z.B. eine vorteilhafte Verwendung in Salben, Gelen und auf Kunststoffoberflächen (in den Oberflächenschichten von Stents), z.B. in Mitteln für die Desinfizierung der Hautoberfläche oder als Fungizid, z.B. für die Füße.

### 2. Herstellung von Cyclotaurolidin ausgehend von Taurolidin

Zu Kontrollzwecken wurde die in Acta Cryst. (1999). C55, 232-234 erwähnte Umsetzung von Taurolidin mit einem Überschuß Methylenglykol (wässrigem Formaldehyd) durchgeführt. Dazu wurden zu einer gerührten Lösung von Formaldehyd (100 ml, 35%, EuAB 5,0) bei Raumtemperatur insgesamt 20 g Taurolidin in 10 Portionen zugegeben. Nach der Zugabe der ersten drei Portionen wurde die Mischung so lange gerührt, bis sie klar wurde. Anschließend wurde die Mischung allmählich auf 55°C erwärmt, und es wurde der Rest des Taurolidins (in 7 Portionen) zugesetzt. Die erhaltene farblose Lösung wurde 10 min gerührt, und dann wurde die Temperatur durch Abkühlen langsam auf 5°C vermindert. Bei 30°C wurde die Lösung trüb. Nach 30 min Rühren der Lösung bei 5°C wurde der gebildete Niederschlag unter Absaugen abfiltriert und mit kaltem Wasser gewaschen. Die Ausbeute des feuchten Rohmaterials betrug 21 g. Nach dem Trocknen und einer Umkristallisation aus Ethanol wurden farblose Kristalle erhalten, die durch Absaugen isoliert wurden.

Ausbeute: 17 g; R_{f} = 0,65 (CHCl₃/MeOH 9.1, Silicagel); Schmelzpunkt 148-149°C. Die Elementaranalyse und die IR- und ¹³C-NMR-Daten bestätigten die Identität des ausgehend von Taurolidin erhaltenen Produkts mit dem Produkt gemäß Beispiel 1.

### 3. Untersuchung der antimikrobiellen Wirksamkeit von wässrigen Cyclotaurolidinlösungen

Orientierende Lösungsversuche von Cyclotaurolidin in Wasser zeigten, dass die Löslichkeit von Cyclotaurolidin in 100 ml Wasser bei Raumtemperatur maximal etwa 1,3 g betrug.

Für die Prüfung auf antimikrobielle Wirksamkeit wurden wässrige Lösungen mit einem Gehalt an Cyclotaurolidin in 100 ml Wasser von 0,85 g verwendet.

Im Hinblick auf eine mögliche Verwendung als Locklösung wurde für die Prüfung auf antimikrobielle Wirksamkeit eine Lösung dadurch hergestellt, dass man 0,85 g Cyclotaurolidin sowie 4,8 g Mononatriumcitrat in Wasser (100 ml) unter Rühren auflöste, wobei eine klare farblose Lösung erhalten wurde. Nachdem der pH der Lösung auf 6,3 eingestellt worden war, wurde die Lösung filtriert und in Ampullen abgefüllt.

Die bakterizide und fungizide Wirksamkeit dieser Lösung wurde von einem anerkannten Testlabor gemäß DIN EN 1040 und DIN EN 1275 (Membranfiltration) gegen die Testorganismen S. Aureus (ATCC 6538), P. Aeruginosa (ATCC 15442), S. Epidermis (ATCC 12288), C. Albicans (ATCC 10231) und A. Niger (ATCC 16404) getestet. Es wurden die für eine Einwirkungszeit der Testlösung von 60 min und von 24 h bei einer Prüftemperatur von 20° ± 1°C beobachtbaren Keimzahlreduktionen ermittelt.

Die Ergebnisse werden in Log-Stufen der Keimzahlreduktion angegeben, wobei in Anlehnung an DIN EN 1040 und DIN EN 1275 gegenüber den Testkeimen S. Aureus, P. Aeruginosa und S. Epidermis Mindestreduktionen von ≥ 5,0 Log-Stufen gefordert werden, und für C. Albicans und A. Niger Log-Stufen von ≥ 4,0.

Nach 60 min wurden die folgenden Keimzahlreduktionen (angegeben in Log-Stufen) erhalten: S. Aureus 2,04, P. Aeruginosa >5,27, S. Epidermis 1,22, C. Albicans >4,26, A. Niger >4,08.

Nach einer Einwirkungszeit von 24 h betrugen die entsprechenden Werte für S. Aureus >5,25, für P. Aeruginosa >5,27, für S. Epidermis >5,29, für C. Albicans >4,26 und für A. Niger >4,08.

Eine Vergleichslösung des Standes der Technik in Form einer 2 Gew.-% Taurolidin sowie eine entsprechende Menge Citrat enthaltenden wässrigen Locklösung vom pH 6,3 lieferte die folgenden entsprechenden Ergebnisse:
- 60 min:: S. Aureus 1,33, P. Aeruginosa >5,27, S. Epidermis >0,99, C. Albicans 0, A. Niger 1,18.
- 24 h:: ≥ 5,25, ≥ 5,27, ≥ 5,29, 3,70, ≥ 4,08.

Ein Vergleich der Ergebnisse zeigt, dass die unter Verwendung von Cyclotaurolidin hergestellte Lösung trotz einer Konzentration, die deutlich unter der üblicher Taurolidinlösungen liegt, unter allen Testbedingungen bessere Keimzahlreduktionen lieferte. Besonders auffällig ist die erheblich höhere Wirksamkeit gegenüber den problematischen Keimen C. Albicans und A. Niger, für die bereits nach 60 min die geforderten Werte erreicht werden, während die bekannte Vergleichslösung bei diesen eukaryontischen Mikroorganismen die geforderten Werte im Falle von A. Niger erst nach 24 h erreicht.

## Patentansprüche

1. Verwendung von 7-Oxa-2-thia-1,5-diazabicyclo[3.3.1]-nonan-2,2-dion ("Cyclotaurolidin") zur Herstellung antimikrobieller Formulierungen.

2. Verwendung nach Anspruch 1 zur Herstellung wässriger antimikrobieller Lösungen für technische oder medizinische Zwecke.

3. Verwendung nach Anspruch 1 oder 2 zur Herstellung von Locklösungen für Katheter und Port-Systeme zur Verhinderung von Infektionen und Sepsis von Patienten, die durch mikrobiell kontaminierte Katheter oder Port-Systeme ausgelöst werden.

4. Verwendung nach Anspruch 3, bei der man eine wässrige Lösung von Cyclotaurolidin mit einer Cyclotaurolidin-Konzentration von 0,5 Gew.-% bis zur Sättigungskonzentration bei Raumtemperatur herstellt, die außerdem ein gelöstes Alkalicitrat und/oder Citronensäure enthält.

5. Wässrige Locklösung für Katheter und Portsysteme in Form einer wässrigen Lösung, die durch Auflösen von Cyclotaurolidin sowie von Mononatriumcitrat in Wasser hergestellt wurde und einen pH im Bereich von 4,5 bis 7,5, vorzugsweise 5,5 bis 7,5, aufweist.

6. Wässrige Locklösung nach Anspruch 5, die außerdem einen oder mehrere weitere Bestandteile enthält, die ausgewählt sind aus Taurinamid, Taurinamidhydrochlorid, einer oder mehreren Substanzen mit antibiotischer Wirksamkeit, einem physiologisch verträglichen Polyol, Polyvinylpyrrolidon, Puffersubstanzen und/oder Heparin.

7. Verwendung einer wässrigen Locklösung nach Anspruch 5 oder 6 zur Füllung von Kathetern oder Port-Systemen, die für die Hämodialyse, für die Medikamentenzufuhr in der Onkologie und Notfallmedizin oder für die parenterale Ernährung von Patienten bestimmt sind, wobei die Katheter oder Port-Systeme für die Zeit, in der die Katheter oder Port-Systeme nicht medizinisch genutzt werden, mit der Locklösung gefüllt werden.

8. Verfahren zur Herstellung von 7-Oxa-2-thia-1,5-diazabicyclo[3.3.1]nonan-2,2-dion ("Cyclotaurolidin"), **dadurch gekennzeichnet, dass** man eine wäßrige Lösung von Taurinamid- in einer Konzentration von über 10 Gew.-%, vorzugsweise etwa 20 Gew.-% oder mehr, zu einer etwa 35%igen Lösung von Formaldehyd in Wasser, die Formaldehyd in einer mindestens 3fachen stöchiometrischen Menge, bezogen auf die Menge des Taurinamids, enthält, zutropft, die erhaltene Reaktionsmischung zur Vervollständigung der Umsetzung erwärmt, danach abkühlt und das erhaltene feste Reaktionsprodukt von der Reaktionslösung abfiltriert sowie gegebenenfalls durch Umkristallisation reinigt.

9. Verfahren nach Anspruch 8, **dadurch gekennzeichnet, dass** man die Reaktionsmischung nach Abschluss der Taurinamid-Zugabe auf eine Temperatur von 55 °C und darüber, insbesondere 60 °C und darüber, erwärmt, in einem erwärmten Zustand für 10 min bis 45 min rührt und dann auf eine Temperatur von weniger als 15 °C, insbesondere etwa 10°C, abkühlt und den sich bildenden Niederschlag sammelt.

## Claims

1. The use of 7-oxa-2-thia-1,5-diazabicyclo[3.3.1]nonane-2,2-dione ("cyclotaurolidine") for the preparation of antimicrobial formulations.

2. The use as claimed in claim 1 for the preparation of aqueous antimicrobial solutions for technical or medical purposes.

3. The use as claimed in claim 1 or 2 for the preparation of lock solutions for catheters and port systems for preventing infections and sepsis of patients which are caused by microbially contaminated catheters or port systems.

4. The use as claimed in claim 3, in which an aqueous solution of cyclotaurolidine having a cyclotaurolidine concentration of from 0.5% by weight to the saturation concentration at room temperature is prepared, which solution also contains a dissolved alkali metal citrate and/or citric acid.

5. An aqueous lock solution for catheters and port systems in the form of an aqueous solution which was prepared by dissolving cyclotaurolidine and monosodium citrate in water and has a pH in the range of from 4.5 to 7.5, preferably from 5.5 to 7.5.

6. The aqueous lock solution as claimed in claim 5, which also contains one or more further constituents which are selected from taurinamide, taurinamide hydrochloride, one or more substances having antibiotic activity, a physiologically acceptable polyol, polyvinylpyrrolidone, buffer substances and/or heparin.

7. The use of an aqueous lock solution as claimed in claim 5 or 6 for filling catheters or port systems which are intended for hemodialysis, for the supply of medicaments in oncology and emergency medicine or for the parenteral nutrition of patients, the catheters or port systems being filled with the lock solution for the time during which the catheters or port systems are not used medically.

8. A process for the preparation of 7-oxa-2-thia-1,5-diazabicyclo[3.3.1]nonane-2,2-dione ("cyclotaurolidine"), **characterized in that** an aqueous solution of taurinamide in a concentration of more than 10% by weight, preferably about 20% by weight or more, is added dropwise to an about 35% solution of formaldehyde in water, which contains formaldehyde in at least 3 times the stoichiometric amount, based on the amount of taurinamide, the reaction mixture obtained is heated to complete the reaction and then cooled and the resulting solid reaction product is filtered off from the reaction solution and, if appropriate, purified by recrystallization.

9. The process as claimed in claim 8, **characterized in that** after the end of the taurinamide addition, the reaction mixture is heated to a temperature of 55°C or higher, in particular 60°C or higher, stirred in a heated state for from 10 min to 45 min and then cooled to a temperature of less than 15°C, in particular about 10°C, and the resulting precipitate is collected.

## Revendications

1. Utilisation de 7-oxa-2-thia-1,5-diazabicyclo (3.3.1) - nonane-2.2-dione (« cyclotaurolidine ») pour la préparation de formulations antimicrobiennes.

2. Utilisation selon la revendication 1 pour la préparation de solutions aqueuses antimicrobiennes à des fins techniques ou médicales.

3. Utilisation selon la revendication 1 ou 2 pour la préparation de solutions verrou pour cathéters et chambres implantables pour éviter chez les patients les infections et les septicémies provoquées par des cathéters ou des chambres implantables de port contaminés par des microbes.

4. Utilisation selon la revendication 3, selon laquelle on prépare une solution aqueuse de cyclotaurolidine avec une concentration de cyclotaurolidine de 0,5 % en poids jusqu'à une concentration de saturation à température ambiante, laquelle comprend en outre un citrate alcalin dissous et / ou de l'acide citrique.

5. Solution verrou aqueuse pour cathéters et chambres implantables sous la forme d'une solution aqueuse, qui a été préparée par dissolution de cyclotaurolidine ainsi que de citrate monosodique dans de l'eau, et qui présente un pH situé dans une plage de 4,5 à 7,5, de préférence de 5,5 à 7,5.

6. Solution verrou aqueuse selon la revendication 5, qui contient en outre un ou plusieurs autres composants, qui sont sélectionnés à partir de taurinamide, de chlorhydrate de taurinamide, d'une ou de plusieurs substances à effet antibiotique, d'un polyol compatible, de polyvinylpyrrolidone, de substances tampon et / ou d'héparine.

7. Utilisation d'une solution verrou aqueuse selon la revendication 5 ou 6 pour le remplissage de cathéters ou de chambres implantables, qui sont destinés à l'hémodialyse, à l'administration de médicaments en oncologie et dans la médecine des urgences, ou pour l'alimentation parentérale de patients, les cathéters ou chambres implantables étant remplis avec la solution verrou pendant le temps au cours duquel lesdits cathéters ou chambres implantables ne sont pas utilisés médicalement.

8. Procédé pour la préparation de 7-oxa-2-thia-1,5 -diazabicyclo (3.3.1) - nonane- 2.2-dione (« cyclotaurolidine »), **caractérisé en ce que** l'on ajoute goutte à goutte une solution aqueuse de taurinamide d'une concentration de plus de 10% en poids, de préférence d'environ 20 % en poids ou plus, à une solution d'environ 35% de formaldéhyde dans de l'eau, qui contient du formaldéhyde en quantité stoechiométrique au moins triple par rapport à la quantité de taurinamide, on chauffe le mélange de réaction jusqu'à la transformation complète, puis on refroidit et on sépare par filtration le produit de réaction solide de la solution de réaction et, le cas échéant, on purifie par recristallisation.

9. Procédé selon la revendication 8, **caractérisé en ce que**, après la fin de l'addition de taurinamide, on chauffe le mélange de réaction à une température de 55°C et plus, en particulier de 60°C et plus, l'agite à l'état chauffé pendant 10 minutes à 45 minutes, et on le refroidit ensuite à une température inférieure à 15°C, en particulier d'environ 10°C, et collecte le précipité qui se forme.
